(19) Europäisches Patentamt
European Patent Office
Office européen des brevets

(11) Veröffentlichungsnummer: **0 349 832**
**A2**

(12) **EUROPÄISCHE PATENTANMELDUNG**

(21) Anmeldenummer: 89111273.2

(22) Anmeldetag: 21.06.89

(51) Int. Cl.[4]: C07D 487/04 , A01N 43/90 , C07D 413/12 , C07D 417/12 , //(C07D487/04,249:00,237:00)

Die Bezeichnung der Erfindung wurde geändert (Richtlinien für die Prüfung im EPA, A-III, 7.3).

(30) Priorität: 04.07.88 JP 165040/88

(43) Veröffentlichungstag der Anmeldung: 10.01.90 Patentblatt 90/02

(84) Benannte Vertragsstaaten: BE CH DE FR GB IT LI NL

(71) Anmelder: NIHON TOKUSHU NOYAKU SEIZO K.K.
Itohpia Nihonbashi Honcho Building 7-1, Nihonbashi Honcho 2-chome
Chuo-ku Tokyo 103(JP)

(72) Erfinder: Kume, Toyohiko
6-7-8, Asahigaoka
Hino-shi Tokyo(JP)
Erfinder: Goto, Toshio
3454-21, Honmachida
Machida-shi Tokyo(JP)
Erfinder: Kamochi, Atsumi
2-24-10, Higashi-Toyoda
Hino-shi Tokyo(JP)
Erfinder: Shibuya, Katsuhiko
39-15, Namiki-cho
Hachioji-shi Tokyo(JP)
Erfinder: Miyauchi, Hiroshi
39-15, Namiki-cho
Hachioji-shi Tokyo(JP)

(74) Vertreter: Schumacher, Günter, Dr. et al
c/o Bayer AG Konzernverwaltung RP Patentabteilung
D-5090 Leverkusen 1 Bayerwerk(DE)

(54) **Triazolo[1,2-a]pyridazinderivate, Verfahren und Zwischenprodukte zu ihrer Herstellung und diese enthaltende Herbizidmittel.**

(57) Offenbart werden heterocyclische Verbindungen der Formel (I)

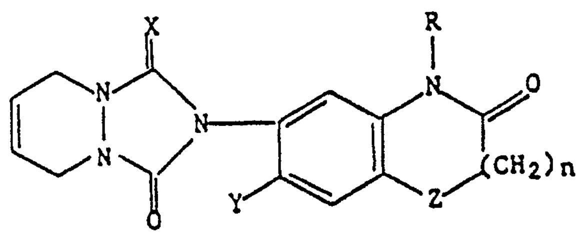

(I)

,

in der
X ein Sauerstoff-Atom oder Schwefel-Atom bezeichnet,
Y ein Wasserstoff-Atom oder Halogen-Atom bezeichnet,
Z ein Sauerstoff-Atom oder Schwefel-Atom bezeichnet,
n 0 oder 1 bezeichnet und
R Wasserstoff, eine Alkyl-Gruppe mit 1 bis 6 Kohlenstoff-Atomen, eine Alkenyl-Gruppe mit 2 bis 4 Kohlenstoff-Atomen oder eine Alkinyl-Gruppe mit 2 bis 4 Kohlenstoff-Atome- bezeichnet,
ein Verfahren zur Herstellung dieser heterocyclischen Verbindungen sowie deren Verwendung als Herbizide.

EP 0 349 832 A2

## Heterocyclische Verbindungen

Die vorliegende Erfindung betrifft neue heterocyclische Verbindungen, ein Verfahren zu ihrer Herstellung und ihre Verwendung als Herbizide.

Es wurde bereits offenbart, daß bestimmte Benzoxazin-Verbindungen als Herbizide von Nutzen sind (siehe die EP-OSen 176 101 und 230 874).

Nunmehr wurden neue heterocyclische Verbindungen der Formel (I)

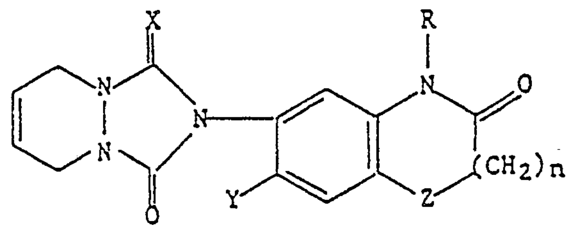

(I)

gefunden, in der

X ein Sauerstoff-Atom oder Schwefel-Atom bezeichnet,
Y ein Wasserstoff-Atom oder Halogen-Atom bezeichnet,
Z ein Sauerstoff-Atom oder Schwefel-Atom bezeichnet,
n 0 oder 1 bezeichnet und
R Wasserstoff, eine Alkyl-Gruppe mit 1 bis 6 Kohlenstoff-Atomen, vorzugsweise mit bis 4 Kohlenstoff-Atomen, eine Alkenyl-Gruppe mit 2 bis 4 Kohlenstoff-Atomen oder eine Alkinyl-Gruppe mit 2 bis 4 Kohlenstoff-Atomen bezeichnet.

Die Alkyl-, Alkenyl- und Alkinyl-Gruppen können geradkettig oder verzweigt sein.

Heterocyclische Verbindungen der Formel (I) werden erhalten, wenn Verbindungen der nachstehenden Formel (II)

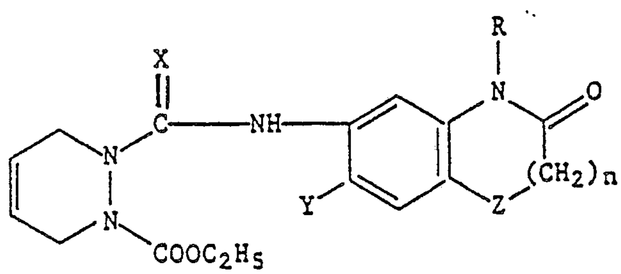

(II)

in der

X, Y, Z, n und R die im Vorstehenden angegebenen Bedeutungen haben, in Gegenwart von Kondensationsmitteln und inerten Lösungsmitteln unter Ringschluß kondensiert werden.

Die neuen heterocyclischen Verbindungen gemäß der vorliegenden Erfindung zeigen potente herbizide Eigenschaften.

Überraschenderweise zeigen die erfindungsgemäßen heterocyclischen Verbindungen eine wesentlich stärkere herbizide Wirkung als die aus dem oben genannten Stand der Technik, beispielsweise den oben genannten EP-OSen 176 101 und 230 874, bekannten Verbindungen sowie eine beständige Bekämpfungswirkung gegen Unkräuter auf höher gelegenen Ackerböden mit Nutzpflanzen, wobei sie gleichzeitig gegenüber diesen Nutzpflanzen gute Verträglichkeit zeigen.

Unter den erfindungsgemäßen heterocyclischen Verbindungen der Formel (I) sind bevorzugte Verbindungen diejenigen, in denen

X ein Sauerstoff-Atom oder Schwefel-Atom ist,
Y ein Wasserstoff-Atom oder Fluor-Atom ist,
Z ein Sauerstoff-Atom oder Schwefel-Atom ist,
n 0 oder 1 ist und
R Wasserstoff, eine Alkyl-Gruppe mit 1 bis 3 Kohlenstoff-Atomen, eine Alkenyl-Gruppe mit 2 bis 3

Kohlenstoff-Atomen oder die Propargyl-Gruppe ist.

Ganz besonders bevorzugte heterocyclische Verbindungen der Formel (I) sind diejenigen, in denen

X ein Sauerstoff-Atom ist,
Y ein Fluor-Atom ist,
Z ein Sauerstoff-Atom oder Schwefel-Atom ist,
n 0 oder 1 ist und
R Methyl, Ethyl, n-Propyl, iso-Propyl, Allyl oder Propargyl ist.

Speziell genannt seien die folgenden Verbindungen:

5,8-Dihydro-2-(6-fluoro-3-propargyl-2-benzothiazolon-5-yl)-1H-[1,2,4]triazolo[1,2-a]pyridazin-1,3(2H)-dion,
5,8-Dihydro-2-(6-fluoro-3-propargyl-2-benzothiazolon-5-yl)-1H-[1,2,4]triazolo[1,2-a]pyridazin-1-thion-3(2H)-on,
5,8-Dihydro-2-(7-fluoro-4-propargyl-4H-1,4-benzoxazin-3(4H)-on-6-yl)-1H-[1,2,4]triazolo[1,2-a]pyridazin-1,3-(2H)-dion,
5,8-Dihydro-2-(7-fluoro-4-propargyl-4H-1,4-benzoxazin-3(4H)-on-6-yl)-1H-[1,2,4triazolo[1,2-a]pyridazin-1-thion-3(2H)-on.

Wenn beispielsweise (2-Ethoxycarbonyl-1,2,3,6-tetrahydropyridazin-1-yl-carbonylamino)-6-fluoro-3-propargyl-benzothiazol-2-on bei der Ringschluß-Reaktion eingesetzt wird, kann der Reaktionsverlauf durch die folgende Gleichung dargestellt werden:

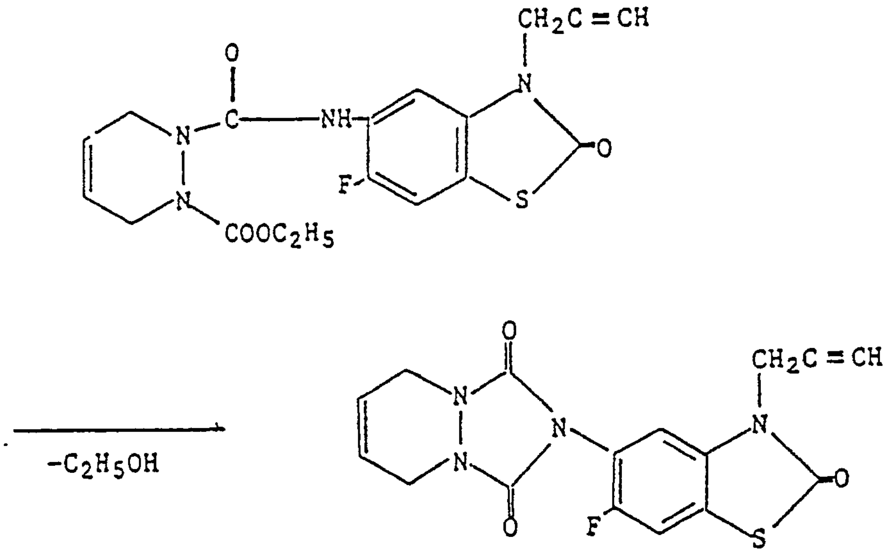


Die Verbindungen der Formel (II) bezeichnen Verbindungen auf der Grundlage der im Vorstehenden gegebenen Definitionen für X, Y, Z, n und R, vorzugsweise solche auf der Grundlage der im Vorstehenden gegebenen bevorzugten Definitionen.

Die Verbindungen der Formel (II) sind neu und können erhalten werden, wenn Verbindungen der Formel (III)

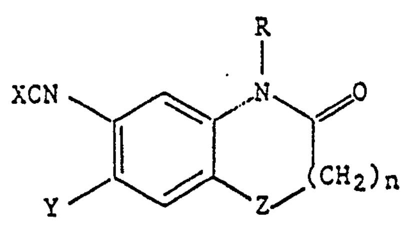

(III)

in der

X, Y, Z, n und R die im Vorstehenden angegebenen Bedeutungen haben, mit 2-Ethoxycarbonyl-1,2,3,6-tetrahydropyridazin in Gegenwart inerter Lösungsmittel wie Toluol umgesetzt werden.

Die Verbindungen der Formel (III) sind bereits bekannt (siehe die JP-OS 152683/1986). Als Beispiele genannt seien

6-Fluoro-5-isocyanato-3-propargylbenzothiazol-2-on, 6-Fluoro-5-isothiocyanato-3-propargylbenzothiazol-2-on und

7-Fluoro-6-isocyanato-4-propargyl-4H-1,4-benzoxazin-3-on.

Als geeignete Verdünnungsmittel für die Durchführung des Verfahrens können beliebige Arten inerter organischer Lösungsmittel genannt werden.

Als Beispiele für diese Verdünnungsmittel eingesetzt werden können Wasser, aliphatische, cycloaliphatische und aromatische Kohlenwasserstoffe, die chloriert sein können oder auch nicht, wie beispielswiese Hexan, Cyclohexan, Petrolether, Ligroin, Benzol, Toluol, Xylol, Methylenchlorid, Chloroform, Kohlenstofftetrachlorid, Ethylenchlorid, Chlorbenzol und weitere, Ether wie beispielsweise Diethylether, Methylethylether, Diisopropylether, Dibutylether, Propylenoxid, Dioxan, Tetrahydrofuran, Ketone wie beispielsweise Aceton, Methylethylketon, Methyl-iso-propylketon, Methyl-isobutylketon, Nitrile wie beispielsweise Acetonitril, Propionitril, Acrylnitril, Alkohole wie Methanol, Ethanol, iso-Propanol, Butanol, Ethylenglycol, Ester wie beispielsweise Ethylacetat, Amylacetat, Säureamide wie beispielsweise Dimethylformamid, Dimethylacetamid, Sulfone und Sulfoxide wie beispielsweise Dimethylsulfoxid, Sulfolan, und Basen wie beispielsweise Pyridin und so weiter.

Bei der Durchführung des Verfahrens ist der Einsatz eines Katalysators bevorzugt oder sogar notwenig, und als Katalysatoren erwähnt seien beispielsweise Natriummethoxid, Natriumethoxid, Natriumhydroxid und Kaliumhydroxid.

Die Reaktionstemperatur des Verfahrens kann innerhalb eines ziemlich breiten Bereichs variiert werden. Im allgemeinen wird die Reaktion bei einer Temperatur von etwa 30 °C bis 150 °C, vorzugsweise bei einer Temperatur von etwa 40 °C bis etwa 90 °C, durchgeführt. Es wird bevorzugt, die Reaktion bei normalem Druck durchzuführen, wenngleich auch ein höherer oder niedrigerer Druck angewandt werden kann.

Bei dem Verfahren kann die angestrebte Verbindung dadurch erhalten werden, daß die Verbindung der allgemeinen Formel (II) beispielsweise einer Kondensationsreaktion unter Ringschluß in einem inerten Lösungsmittel wie Methanol und in Gegenwart einer katalytischen Menge Natriummethoxid unterworfen wird.

Die erfindungsgemäßen aktiven Verbindungen können als Entlaubungsmittel, Abbrandmittel, Mittel zur Zerstörung breitblättriger Pflanzen und insbesondere als Unkrautvernichtungsmittel eingesetzt werden. Unter "Unkräutern" im weitesten Sinne sind alle Pflanzen zu verstehen, die an Stellen wachsen, wo sie nicht erwünscht sind. Ob die erfindungsgemäßen Substanzen als totale oder selektive Herbizide wirken, hängt im wesentlichen von der verwendeten Menge ab.

Die aktiven Verbindungen gemäß der Erfindung können beispielsweise in Verbindung mit den folgenden Pflanzen verwendet werden:

Dikotyledonen-Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver und Centaurea. Dikotyledonen-Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita. Monokotyledonen-Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera. Monokotyledonen-Kulturen der Gattungen:

Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleicher Weise.

In Abhängigkeit von den Konzentrationen können die Verbindungen zur totalen Bekämpfung von Unkräutern, beispielsweise auf Industriegelände und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in Jahreskulturen verwendet werden.

Die Wirkstoffe können in übliche Präparate wie Lösungen, Emulsionen, benetzbare Pulver, Suspensionen, Pulver, Schäume, Pasten, Granulat, Aerosole, mit dem Wirkstoff imprägnierte natürliche und synthetische Materialien, sehr feine Kapseln in polymeren Substanzen, Beschichtungsmittel zum Aufbringen auf das Saatgut und Formulierungen, die in Verbindung mit Verbrennungseinrichtungen wie Räucherpatronen, Räucherdosen und Räucherschlangen eingesetzt werden, sowie ULV-Kaltvernebelungs- und Warmvernebelungspräparate umgewandelt werden.

Diese Präparate können in bekannter Weise hergestellt werden, beispielsweise durch Vermischen der Wirkstoffe mit Streckmitteln, d.h. flüssigen oder verflüssigten gasförmigen oder festen Verdünnungsmitteln oder Trägern, gegebenenfalls unter Verwendung von grenzflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorobenzole, Chloroethylene oder Methylenchlorid, aliphatische oder alicyclische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Unter verflüssigten gasförmigen Verdünnungsmitteln oder Trägern sind Flüssigkeiten zu verstehen, die bei normaler Temperatur und unter normalem Druck gasförmig sein würden, beispielsweise Aerosol-Treibmittel wie halogenierte Kohlenwasserstoffe sowie Butan, Propan, Stickstoff und Kohlenstoffdioxid.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie Polyoxyethylen-Fettsäureester, Polyoxyethylen-Fettalkoholether, beispielsweise Alkylarylpolyglycolether, Alkylsulfonate, Alkylsulfate, Arylsulfonate sowie Hydrolyseprodukte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z.B. Gummi arabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe oder Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,1 bis 95 Gewichts-%, vorzugsweise von 0,5 bis 90 Gewichts-% des Wirkstoffs.

Die Wirkstoffe gemäß der vorliegenden Erfindung können als solche oder in Form ihrer Präparate auch zur Unkrautbekämpfung als Mischungen mit anderen Herbiziden eingesetzt werden, wobei Fertigpräparate oder Tankmischungen möglich sind.

Mischungen mit anderen aktiven Verbindungen wie Herbiziden, Fungiziden, Insektiziden, Akariziden, Nematiziden, vogelabweisenden Mitteln, Pflanzennährstoffen sowie Mitteln zur Verbesserung der Bodenstruktur sind ebenfalls möglich.

Die Wirkstoffe können als solche, in Form von Präparaten oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie werden in üblicher Weise angewandt, beispielsweise durch Bewässern, Sprühen, Zerstäuben oder Streuen.

Die erfindungsgemäßen Wirkstoffe können vor oder nach dem Auflaufen der Pflanzen zur Anwendung gelangen.

Sie können auch vor der Einsaat in den Boden eingearbeitet werden. Insbesondere werden sie nach dem Auflaufen der Pflanzen angewandt.

Die eingesetzten Mengen der aktiven Verbindung können innerhalb eines Bereichs von erheblicher Breite variiert werden. Sie hängen wesentlich von der Art der angestrebten Wirkung ab. Im allgemeinen liegen die aufgewandten Mengen des Wirkstoffs zwischen 0,0001 und 2 kg/ha, vorzugsweise zwischen 0,001 und 1 kg/ha.

Die Herstellung und Verwendung der erfindungsgemäßen Verbindungen sind den folgenden Beispielen zu entnehmen.

<u>Herstellungsbeispiele:</u>

Beispiel 1:

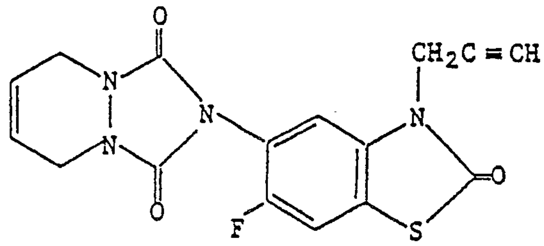

2 g 6-Fluoro-5-isocyanato-3-propargyl-2-benzothiazolon wurden in 20 ml Toluol gelöst, und zu der Lösung wurden tropfenweise 1,32 g 2-Ethoxycarbonyl-1,2,3,6-tetrahydropyridazin bei Raumtemperatur hinzugefügt. Anschließend wurde 6 h bei Raumtemperatur gerührt, und das Lösungsmittel wurde bei vermindertem Druck abdestilliert.

Der Rückstand wurde mit 60 ml Methanol und 10 mg Natriummethoxid versetzt, und die Mischung wurde 4 h unter Rückfluß erhitzt. Die Reaktionsmischung wurde 30 min bei einer Temperatur im Bereich von 10 °C bis 20 °C gerührt, und der resultierende feste Stoff wurde daraus abfiltriert, mit Methanol und Toluol gewaschen und getrocknet, wonach 1,2 g des angestrebten 5,8-Dihydro-2-(6-fluoro-3-propargyl-2-benzothiazolon-5-yl)-1H-[1,2,4]triazolo[1,2-a]pyridazin-1,3(2H)-dions mit einem Schmelzpunkt von 274-278 °C erhalten wurde.

An Stelle des 6-Fluoro-5-isocyanato-3-propargyl-2-benzothiazolons wurden in dem vorstehenden Beispiel 1 6-Fluoro-5-isothiocyanato-3-propargyl-2-benzothiazolon oder 7-Fluoro-6-isocyanato-4-propargyl-4H-1,4-benzoxazin-3-on eingesetzt, so daß 5,8-Dihydro-2-(6-fluoro-3-propargyl-2-benzothiazolon-5-yl)-1H-[1,2,4]triazolo[1,2-a]pyridazin-1-thion-3(2H)-on bzw. 5,8-Dihydro-2-(7-fluoro-4-propargyl-4H-1,4-benzoxazin-3-(4H)-on-6-yl)-1H-[1,2,4]triazolo[1,2-a]pyridazin-1,3(2H) -dion erhalten wurden.

In der nachstehenden Tabelle 1 sind die Verbindungen aufgeführt, die nach dem gleichen Verfahren, das in Beispiel 1 angewandt wurde, hergestellt werden können, zusammen mit den Verbindungen, die tatsächlich nach den Beispielen gemäß der vorliegenden Erfindung hergestellt worden sind.

## Tabelle 1

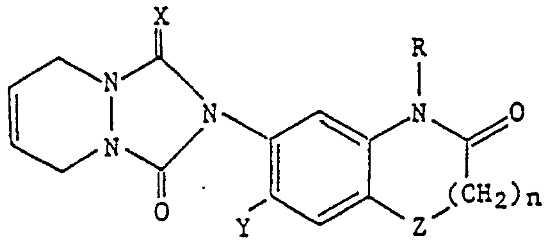

| Verbindung Nr. | X | Y | Z | n | R | Schmp. (°C) |
|---|---|---|---|---|---|---|
| 1 | O | F | S | 0 | $CH_3$ | |
| 2 | O | F | S | 0 | $C_2H_5$ | |
| 3 | O | F | S | 0 | $C_3H_7$-n | |
| 4 | O | F | S | 0 | $CH_2CH=CH_2$ | |
| 5 | O | F | S | 0 | $CH_2C\equiv CH$ | 274 - 278 |
| 6 | S | F | S | 0 | $CH_2C\equiv CH$ | 253 - 257 |
| 7 | O | F | O | 1 | H | > 300 |
| 8 | O | F | O | 1 | H | 293 - 298 |
| 9 | O | F | O | 1 | $CH_3$ | |
| 10 | O | F | O | 1 | $C_2H_5$ | |
| 11 | O | F | O | 1 | $C_3H_7$-n | |
| 12 | O | F | O | 1 | $CH_2CH=CH_2$ | |
| 13 | O | F | O | 1 | $CH_2C\equiv CH$ | 258 - 262 |
| 14 | S | F | O | 1 | $CH_2C\equiv CH$ | 231 - 235 |
| 15 | O | H | O | 1 | $CH_2CH=CH_2$ | |
| 16 | S | H | O | 1 | $CH_2CH=CH_2$ | |
| 17 | O | H | O | 1 | $CH_2C\equiv CH$ | |
| 18 | S | H | O | 1 | $CH_2CH\equiv CH$ | |

Bezugsbeispiel (Herstellung einer Ausgangs-Verbindung)

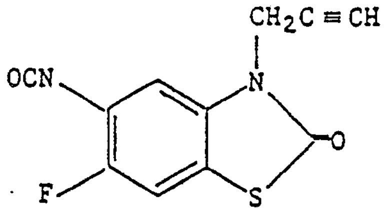

Zu einer aus 3,2 g 5-Amino-6-fluoro-3-propargyl-2-benzothiazolon und 50 g Dioxan bestehenden Lösung wurden tropfenweise 2,4 g Trichloromethylchlorocarbonat bei einer Temperatur im Bereich von 5 °C bis 15 °C hinzugefügt, und die resultierende Mischung wurde 6 h unter Rückfluß erhitzt. Ein Rückstand wurde nach Abdestillieren einer niedrig-siedenden Substanz unter vermindertem Druck erhalten und in 100 ml getrocknetem Toluol gelöst und filtriert. Aus dem erhaltenen Filtrat wurde das Toluol abdestilliert, wonach 3,6 g 6-Fluoro5-isocyanato-3-propargyl-2-benzothiazolon mit einem Schmelzpunkt im Bereich von 134-135 °C erhalten wurden.

Biologische Test-Beispiele

Vergleichs-Verbindung (E-1)

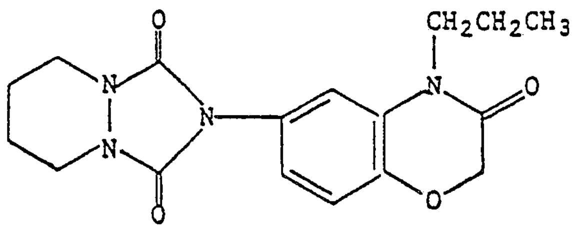

(offenbart in der EP-OS 176 101 / Verbindung Nr. 2)

Beispiel 2

Test gegen Unkräuter in einem überfluteten Reisfeld durch Wasseroberflächen-Anwendung

Herstellung eines Wirkstoff-Präparats

Träger: 5 Gewichtsteile Aceton; Emulgator: 1 Gewichtsteil Benzyloxypolyglycolether.

Ein Wirkstoff-Präparat in Form eines emulgierbaren Konzentrats wurde erhalten durch Vermischen von 1 Gewichtsteil der aktiven Verbindung mit den oben angegebenen Mengen des Trägers und des emulgierenden Mittels. Eine vorher festgelegte Menge des Präparats wurde mit Wasser verdünnt.

Test-Verfahren

Reis-Kulturboden wurde in Töpfe (5 $dm^2$; 25 cm x 20 cm x 9 cm) gefüllt, und Reis-Setzlinge (Varietät: "Nihonbare") im 2,5-Blattstadium (15 cm hoch) wurden jeweils an zwei Stellen pro Topf als Stock von drei Setzlingen umgepflanzt. Samen von Hühnerhirse (Echinochloa oryzicola Vasing.), Schirmkraut (Cyperus difformis L.), Monochoria (Monochoria vaginalis) sowie einjährigen breitblättrigen Unkräutern [Falsche

Pimpernelle (Lindernia pyxidaria L.), Rotala indica, Amerikanischer Wasserwurz (Elatine triandra), Rotstengel Ammannia multiflora Roxburgh) und Dopatrium junceum Hamilton] wurden eingesät, und die Töpfe wurden feucht gehalten. Zwei Tage später wurden die Töpfe bis zu einer Tiefe von etwa 2 bis 3 cm überflutet. Fünf Tage nach dem Umpflanzen der Setzlinge wurde die Verbindung der vorliegenden Erfindung, in Form eines wie im Vorstehenden hergestellten emulgierbaren Konzentrats, mittels einer Pipette in einer vorher festgelegten Menge auf die Oberfläche des Wassers aufgebracht. Danach wurde der Zustand der etwa 3 cm tiefen Überflutung aufrechterhalten, und vier Wochen nach der chemischen Behandlung wurden die herbizide Wirkung und die Phytotoxizität gegenüber Reis untersucht und mit Hilfe der folgenden Skala von 0 bis 5 bewertet:

| Bewertung | Herbizide Wirkung (bewertet als Unkrautvernichtungs-Verhältnis, bezogen auf eine unbehandelte Fläche) |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |
| Bewertung | Phytotoxizität gegenüber Reis (bewertet unter Bezug auf die unbehandelte Fläche) |
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

Die Test-Ergebnisse sind anhand typischer Beispiele in Tabelle 2 aufgeführt.

Tabelle 2

| Verbindung Nr. | Wirkstoff-Menge kg/ha | **Herbizide Wirkung** | | | | Phytoxizität gegen Reispflanzen |
|---|---|---|---|---|---|---|
| | | Hühnerhirse | Schirmkraut | Monochoria | Einjähr. breitblättrige Unkräuter | |
| 5 | 0,125 | 4 | 5 | 5 | 5 | 1 |
| | 0,06 | 3 | 5 | 5 | 5 | 0 |
| 13 | 0,125 | 5 | 5 | 5 | 5 | 2 |
| | 0,06 | 4 | 5 | 5 | 5 | 0 |
| Kontroll-Verbindung | | | | | | |
| E-1 | 0,25 | 2 | 3 | 3 | 3 | 3 |
| | 0,125 | 1 | 3 | 2 | 2 | 2 |

Beispiel 3

Test gegen Unkräuter auf höher gelegenem Ackerboden durch Bodenbehandlung vor dem Auflaufen:

In einem Gewächshaus wurden Sojabohnen-Samen in Töpfe von 500 $cm^2$ Fläche, die mit Ackererde gefüllt waren, eingesät, und Erde, die Samen von Hühnerhirse (Echinocloa crus-galli), wilder Melde

(Amaranthus blitum L.) und Gänsefuß (Chenopodium album L.) enthielt, wurde über die Erde in den Töpfen in einer Tiefe von 1 cm gestreut.

Einen Tag nach der Einsaat wurde eine Test-Chemikalie in einer vorher festgelegten Konzentration, hergestellt wie in Beispiel 2, gleichmäßig über die Oberflächenschicht des Bodens in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 2 geprüft. Die Ergebnisse sind anhand typischer Beispiele in Tabelle aufgeführt.

Tabelle 3

| Verbindung Nr. | Wirkstoff-Menge kg/ha | **Herbizide Wirkung** | | | Phytoxizität gegen Sojabohnenpflanzen |
|---|---|---|---|---|---|
| | | Hühnerhirse | Wilde Melde | Gänsefuß | |
| 5 | 0,125 | 5 | 5 | 5 | 1 |
| | 0,06 | 4 | 5 | 5 | 0 |
| 13 | 0,125 | 5 | 5 | 5 | 1 |
| | 0,06 | 4 | 5 | 5 | 0 |
| Kontroll-Verbindung | | | | | |
| E-1 | 0,25 | 1 | 3 | 3 | 2 |
| | 0,125 | 0 | 1 | 2 | 1 |

Beispiel 4

Test gegen Unkräuter auf höher gelegenem Ackerboden durch Laub-Behandlung:

In einem Gewächshaus wurden Mais-Samen in Töpfe von 500 $cm^2$ Fläche, die mit Ackererde gefüllt waren, eingesät, und Erde, die Samen von Fingergras (Digitaria sanguinalis), wilder Melde (Amaranthus blitum L.) und Gänsefuß (Chenopodium album L.) enthielt, wurde über die Erde in den Töpfen in einer Tiefe von 1 cm gestreut.

Nach der Einsaat wurden die Pflanzen 14 Tage angezogen, und eine Test-Chemikalie in einer vorher festgelegten Konzentration, hergestellt wie in Beispiel 2, wurde gleichmäßig über Test-Pflanzen in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 2 geprüft. Die Ergebnisse sind anhand typischer Beispiele in Tabelle aufgeführt.

Tabelle 4

| Verbindung Nr. | Wirkstoff-Menge kg/ha | **Herbizide Wirkung** | | | Phytoxizität gegen Maispflanzen |
|---|---|---|---|---|---|
| | | Fingergras | Wilde Melde | Gänsefuß | |
| 5 | 0,06 | 5 | 5 | 5 | 1 |
| | 0,03 | 5 | 5 | 5 | 0 |
| 13 | 0,06 | 5 | 5 | 5 | 1 |
| | 0,03 | 5 | 5 | 5 | 0 |
| Kontroll-Verbindung | | | | | |
| E-1 | 0,125 | 2 | 3 | 3 | 3 |
| | 0,06 | 1 | 2 | 2 | 2 |

## Ansprüche

1. Heterocyclische Verbindungen der Formel (I)

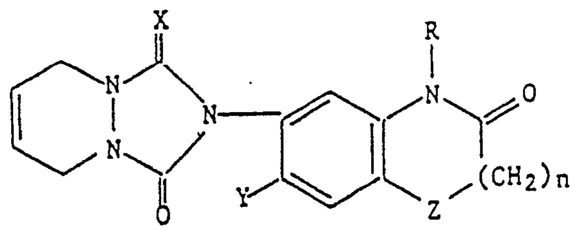

(I)

,

in der
X ein Sauerstoff-Atom oder Schwefel-Atom bezeichnet,
Y ein Wasserstoff-Atom oder Halogen-Atom bezeichnet,
Z ein Sauerstoff-Atom oder Schwefel-Atom bezeichnet,
n 0 oder 1 bezeichnet und
R Wasserstoff, eine Alkyl-Gruppe mit 1 bis 6 Kohlenstoff-Atomen, eine Alkenyl-Gruppe mit 2 bis 4 Kohlenstoff-Atomen oder eine Alkinyl-Gruppe mit 2 bis 4 Kohlenstoff-Atomen bezeichnet.

2. Verbindungen der Formel (I) nach Anspruch 1, worin
X ein Sauerstoff-Atom oder Schwefel-Atom bezeichnet,
Y ein Wasserstoff-Atom oder Fluor-Atom bezeichnet,
Z ein Sauerstoff-Atom oder Schwefel-Atom bezeichnet,
n 0 oder 1 bezeichnet und
R Wasserstoff, eine Alkyl-Gruppe mit 1 bis 3 Kohlenstoff-Atomen, eine Alkenyl-Gruppe mit 2 bis 3 Kohlenstoff-Atomen oder die Propargyl-Gruppe bezeichnet.

3. Verbindungen der Formel (I) nach Anspruch 1, worin
X ein Sauerstoff-Atom bezeichnet,
Y ein Fluor-Atom bezeichnet,
Z ein Sauerstoff-Atom oder Schwefel-Atom bezeichnet,
n 0 oder 1 bezeichnet und
R Methyl, Ethyl, n-Propyl, iso-Propyl, Allyl oder Propargyl bezeichnet.

4. Verbindungen der Formel (I) nach den Ansprüchen 1 bis 3, nämlich
5,8-Dihydro-2-(6-fluoro-3-propargyl-2-benzothiazolon-5-yl)-1H-[1,2,4]triazolo[1,2-a]pyridazin-1,3(2H)-dion

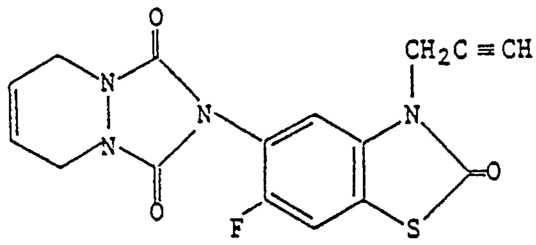

,

5,8-Dihydro-2-(6-fluoro-3-propargyl-2-benzothiazolon-5-yl)-1H-[1,2,4]triazolo[1,2-a]pyridazin-1-thion-3(2H)-on

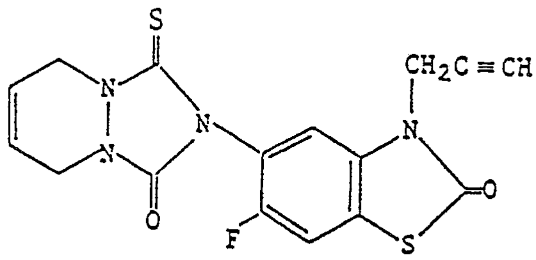

,

5,8-Dihydro-2-(7-fluoro-4-propargyl-4H-1,4-benzoxazin-3(4H)-on-6-yl)-1H-[1,2,4]triazolo[1,2-a]pyridazin-1,3-(2H)-dion

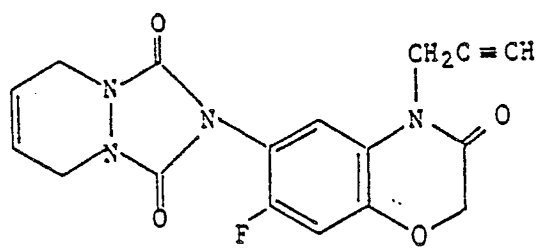

und

5,8-Dihydro-2-(7-fluoro-4-propargyl-4H-1,4-benzoxazin-3(4H)-on-6-yl)-1H-[1,2,4]triazolo[1,2-a]pyridazin-1-thion-3(2H)-on

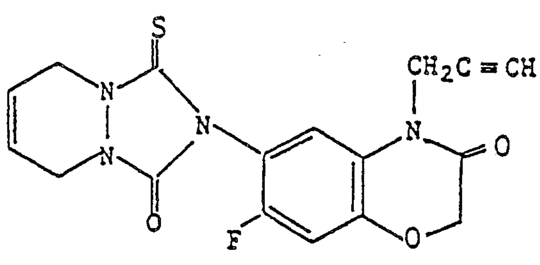

.

5. Verfahren zur Herstellung heterocyclischer Verbindungen der Formel (I)

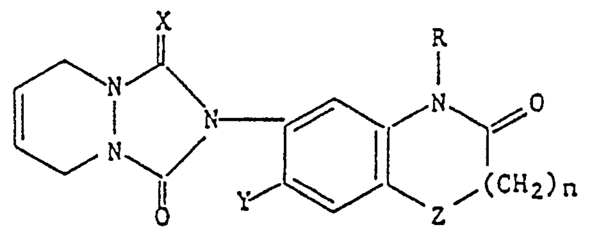

(I)

,

in der
X ein Sauerstoff-Atom oder Schwefel-Atom bezeichnet,
Y ein Wasserstoff-Atom oder Halogen-Atom bezeichnet,
Z ein Sauerstoff-Atom oder Schwefel-Atom bezeichnet,
n 0 oder 1 bezeichnet und
R Wasserstoff, eine Alkyl-Gruppe mit 1 bis 6 Kohlenstoff-Atomen, eine Alkenyl-Gruppe mit 2 bis 4 Kohlenstoff-Atomen oder eine Alkinyl-Gruppe mit 2 bis 4 Kohlenstoff-Atomen bezeichnet,
dadurch gekennzeichnet, daß
Verbindungen der Formel (II)

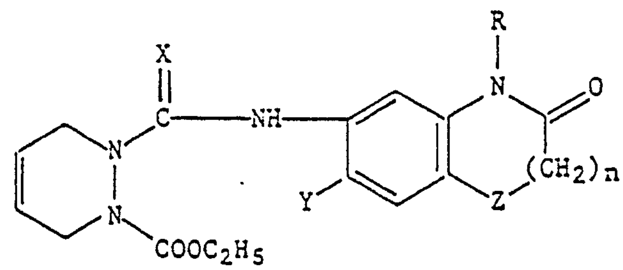

(II)

,

in der
X, Y, Z, n und R die im Vorstehenden angegebenen Bedeutungen haben, in Gegenwart von Kondensationsmitteln und inerten Lösungsmitteln unter Ringschluß kondensiert werden.

6. Herbizide Zusammensetzungen, dadurch gekennzeichnet, daß sie wenigstens eine heterocyclische Verbindung der Formel (I) nach irgendeinem der Ansprüche 1 bis 4 enthalten.

7. Verfahren zur Unkrautbekämpfung, dadurch gekennzeichnet, daß man heterocyclische Verbindungen der Formel (I) nach irgendeinem der Ansprüche 1 bis 4 auf Unkräuter und/oder ihren Lebensraum einwirken läßt.

8. Verwendung heterocyclischer Verbindungen der Formel (I) nach irgendeinem der Ansprüche bis 4 zur Unkrautbekämpfung.

9. Verfahren zur Herstellung herbizider Zusammensetzungen, dadurch gekennzeichnet, daß heterocyclische Verbindungen der Formel (I) nach irgendeinem der Ansprüche 1 bis 4 mit Streckmitteln und/oder grenzflächenaktiven Mitteln vermischt werden.

10. Verbindung der Formel (II)

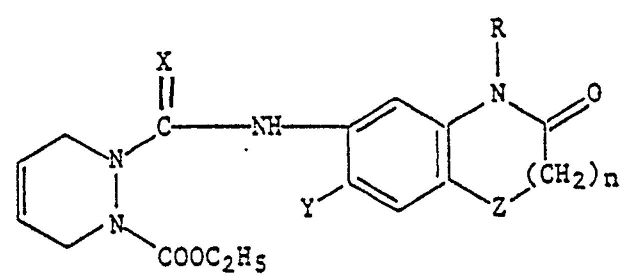

(II)

,

in der

X ein Sauerstoff-Atom oder Schwefel-Atom bezeichnet,

Y ein Wasserstoff-Atom oder Halogen-Atom bezeichnet,

Z ein Sauerstoff-Atom oder Schwefel-Atom bezeichnet,

n 0 oder 1 bezeichnet und

R Wasserstoff, eine Alkyl-Gruppe mit 1 bis 6 Kohlenstoff-Atomen, eine Alkenyl-Gruppe mit 2 bis 4 Kohlenstoff-Atomen oder eine Alkinyl-Gruppe mit 2 bis 4 Kohlenstoff-Atomen bezeichnet.